# EUROPEAN PATENT SPECIFICATION

(11) **EP 3 257 467 B1**
(45) Date of publication and mention of the grant of the patent: **15.05.2019**
(21) Application number: 17180872.8
(22) Date of filing: 29.12.2004
(51) Int. Cl.: A61C 3/00, A61C 5/00, A61N 1/30, A61Q 11/00

(54) **ION EXCHANGE DENTAL DEVICE**
IONENAUSTAUSCH-DENTALVORRICHTUNG
DISPOSITIF DENTAIRE ÉCHANGEUR D'IONS

(30) Priority: 29.12.2003 US 532570 P
(43) Date of publication of application: 20.12.2017
(62) Divisional of application: 04806705.2
(73) Proprietor: Colgate-Palmolive Company, New York, NY 10022 (US)
(72) Inventor: JUBEH, Maher, 97200 Jerusalem (IL); KHAWALED, Kamal, 20200 Shfaram (IL)
(74) Representative: Thum, Bernhard

(56) References cited:
- EP-A1- 1 525 857
- WO-A1-00/29063
- JP-A- H1 080 324
- US-A- 3 215 139
- US-A1- 2002 177 107
- US-A1- 2003 003 421

## Description

### FIELD AND BACKGROUND OF THE INVENTION

The present invention relates to an ion exchange dental device and method and more particularly, to a device and method for improved fluoride uptake by the teeth during treatment for prevention of dental caries.

Dental caries, or tooth decay, is the progressive loss of tooth mineral, followed by bacterial invasion into the de-mineralized tooth. Dental caries is a relatively complex disease. There is abundant evidence that the initiation of caries requires a relatively high proportion of *mutans streptococci* within dental plaque. These bacteria adhere well to the tooth surface, produce higher amounts of acid from sugars than other bacterial types, can survive better than other bacteria in an acid environment, and produce extra-cellular polysaccharides from sucrose. When the proportion of *s. mutans* in plaque is high (in the range of 2-10%), a patient is at high risk for caries. When the proportion is low (less than 0.1%), the patient is at low risk. Two other types of bacteria are also associated with the progression of caries through dentin. These are several species of *lactobacillus,* and *actinomyces viscosus.* These bacteria are highly acidogenic and survive well in acid conditions.

Moreover, diet greatly influences dental caries. Dietary sucrose changes both the thickness and the chemical nature of plaque. *Mutans streptococci* and some other plaque bacteria use the monosaccharide components (glucose and fructose) and the energy of the disaccharide bond of sucrose to assemble extra-cellular polysaccharides. A diet with a high proportion of sucrose therefore increases caries risk.

The mineral of enamel, cementum and dentin is a highly-substituted calcium phosphate salt called apatite. The apatite of newly-formed teeth is rich in carbonate, has relatively little fluoride and is relatively soluble. Cycles of partial demineralization and then remineralization in a fluoride-rich environment creates apatite with less carbonate and more fluoride, and is less soluble. Fluoride-rich, low carbonate apatite can be up to ten times less soluble than apatite low in fluoride and high in carbonate. Topical fluoride also inhibits acid production by plaque bacteria. Fluoride in food and drinks, in dentifrices, oral rinses and gels and in filling materials can therefore all reduce the solubility of teeth, helping to reduce caries risk. These effects are very beneficial, but the amounts of fluoride that can be added to the diet or used topically are limited by safety considerations. High levels of dietary fluoride can cause mottling of tooth enamel during tooth formation, while swallowing high levels can cause symptoms of poisoning.

Attempts at reducing the concentration of fluoride and/or the amount of time of exposure to fluoride have been made. For example, some prior art devices have used an electrical current to stimulate ion exchange, wherein fluoride ions can be incorporated into the teeth, thereby enhancing resistance to caries formation. However, the prior art devices have a circuit which runs through the body of the individual, and are thus classified as iontophoretic devices. The iontophoretic nature of such devices tends to result in insufficient fluoride uptake, largely due to electrochemical polarization that occurs in the body due to the presence of potassium in the blood and bones, which causes a high resistance to fluoride ion flow, thus hampering fluoride uptake.
US 2002/0177107 A1 discloses a toothbrush with a dry cell battery enclosed within the dielectric handle of the brush with two dielectrode plates exposed to facilitate electrolysis of a slurry of saliva containing gels, dentifrice, and rinses.

There is thus a widely recognizes need for, and it would be highly advantageous to have, an ion exchange dental device and method devoid of the above limitations.

### SUMMARY OF THE INVENTION

According to one aspect not covered by the invention, there is provided a method for treating a tooth. The method includes applying a metal salt solution to a tooth, applying an ionizable substance to the tooth, and applying a current flow to the tooth so as to ionize the ionizable substance.

According to another aspect not covered by the invention there is provided for pre-treatment of teeth. The device includes a tray having an upper portion and a lower portion, a sealing lip on the upper portion for enabling close contact between the device and the gums (for sealing), and an absorbent material in the lower portion for receiving an activation solution.

According to an aspect of the present invention there is provided a device for dental treatment. The device includes an applicator for applying a substance to a tooth, the applicator having a first end and a second end, a first electrode attached to a first end of the applicator, a second electrode attached to the second end of the applicator, wherein the first electrode and the second electrode are configured to produce a current flow through the applicator, and an ionizable substance for placement within the applicator, wherein the ionizable substance is configured to undergo ionization upon application of the current flow through the substance, characterized in that said applicator is a dental tray.

According to further features in preferred embodiments of the invention described below, the activation is achieved by using a metal salt solution such as silver nitrate, palladium hydroxide, palladium chloride, copper chloride, or any other suitable solution. In a preferred embodiment, the step of applying a metal salt solution precedes the other steps. In one embodiment, the tooth is rinsed with distilled water after the metal salt solution is applied. In a preferred embodiment, the applying of the metal salt solution is done using a pre-treatment tray which is designed to prevent leakage of the metal salt solution into the mouth. The ionizable substance includes a fluoride compound, preferably sodium fluoride, lithium fluoride, amino fluoride, tin fluoride, or any other suitable fluoride ion donor. The current flow is applied at a voltage within a range of 1-12 volts, and more preferably within 3 to 9 volts. The current may be applied via power supply, rechargeable battery, or a disposable battery embedded within a device.

According to further features in preferred embodiments of the invention, the pre-treatment device further includes an adhesive material placed on the sealing lip for further enabling of close contact between the device and the gums. Alternatively or in addition to the adhesive material, the tray itself may be comprised of heatable plastic material which can be formed onto the teeth and gums during application. The activation solution is a metal salt solution, preferably one of the following: silver nitrate, palladium hydroxide, palladium chloride or copper chloride, but may include any suitable metal salt solution.

According to the invention described below, the applicator is a dental tray . The ionizable substance is a fluoride compound, such as sodium fluoride, lithium fluoride, tin fluoride, amino fluoride, or a combination of fluoride compounds. In the case of a dental tray, the first end is a back curved wall and the first electrode is an anode, which is preferably a flat metal strip. Spacers made of biocompatible plastic are placed on the anode to separate the anode from the tooth. The second end is a front wall and the second electrode is a cathode having a spring mechanism. Preferably, three cathodes are present, each of which is configured to contact a separate tooth, wherein the contact area is minimized. The dental tray may include a sealing lip. In one embodiment, the device includes a power supply, which can be an external electrical power source, a rechargeable battery, or a disposable battery embedded in the device.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
Fig. 1 is a prior art iontophoretic dental device;
Fig. 2 is a prior art iontophoretic toothbrush;
Fig. 3 is an illustration of a pretreatment tray;
Fig. 4 is an isometric view of a device in accordance with a preferred embodiment of the present invention;
Figs. 5a and 5b are cross-sectional illustrations of the device of Fig. 4;
Figs. 6a and 6b are diagrammatic and cross-sectional views of an electric dental device in accordance with another embodiment of the present invention;
Figs. 7a and 7b are diagrammatic and cross-sectional views of an electric dental device in accordance with yet another embodiment of the present invention;
Fig. 8 is a view of an electric toothbrush in accordance with another embodiment not covered by the present invention; and
Fig. 9 is a bar graph illustration of results of using a method in accordance with a preferred embodiment of the present invention.

### DESCRIPTION OF THE PREFERRED EMBODIMENTS

The present invention is of a system and method for enhanced fluoride treatment of the teeth. Specifically, the present invention can be used to increase the uptake of fluoride and/or decrease the uptake of plaque by application of a preparatory solution followed by application of a treatment fluoride-enriched gel and the application of an electrical current directly to the teeth.

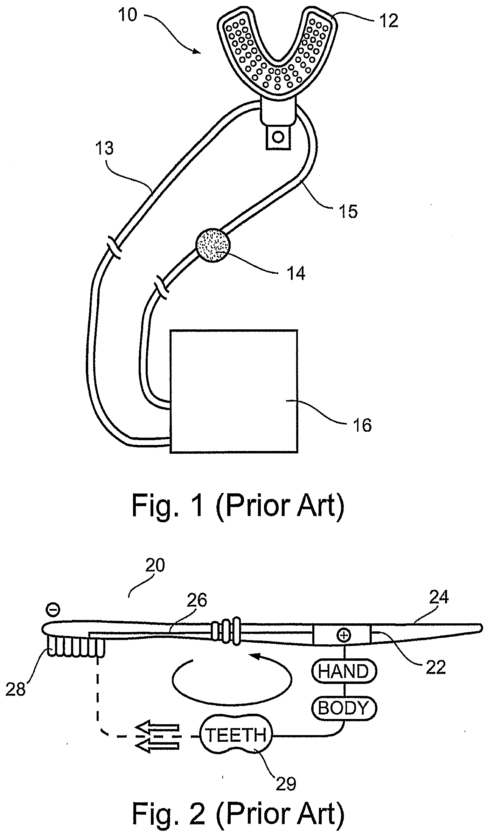

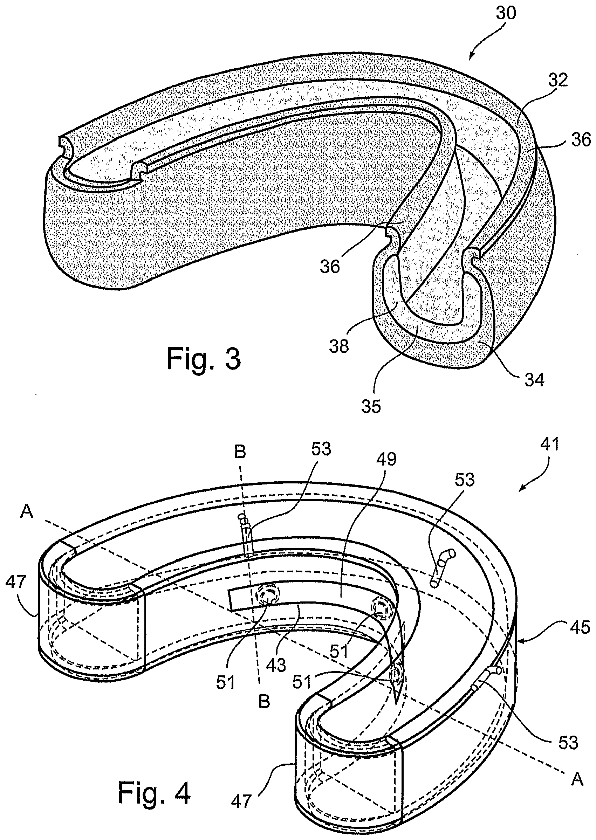

For purposes of better understanding the present invention, as illustrated in Figures 3-8 of the drawings, reference is first made to the construction and operation of prior art iontophoretic dental devices as illustrated in Figs. 1 and 2.

As shown in Fig. 1, a prior art system 10 for iontophoretic uptake of fluoride includes a metallic tray 12, which acts as a first (negative) electrode, connected with a first wire 13 to a power supply 16, and connected with a second wire 15 to a second (positive) electrode 14, which is further connected to power supply 16. Second electrode 14 is designed to be in contact with a location on a body of an individual undergoing the procedure provided herein. . An ionizable form of fluoride, such as lithium fluoride is incorporated in a gel matrix and placed within metallic tray 12. Metallic tray 12 is coated with a non-conducting material, or alternatively, the tray itself comprises non-conducting material, and includes thereon the first electrode. In either case, the tray filled with the fluoride gel is placed in contact with the teeth of the individual. A current is applied via power supply 16, causing ionization of the fluoride and thereby providing fluoride ions in a form that can be assimilated into the tooth structure. The circuit for ionization to occur is completed through the body of the individual, thus classifying it as an iontophoretic device.

It has been shown that in systems such as the one depicted in Fig. 1, in which the circuit runs through the body of the individual, fluoride uptake is insufficient. This result is likely due to electrochemical polarization that occurs in the body due to the presence of potassium in the blood and bones, which causes a high resistance to fluoride ion flow, thus hampering fluoride uptake.

Reference is now made to Fig. 2, which is an illustration of a prior art ionic toothbrush 20. As shown in Fig. 2, ionic toothbrush 20 has a battery 22 located on a handle 24 of ionic toothbrush 20. A metal rod 26 runs through the body of ionic toothbrush 20 and into the area of bristles 28. When battery 22 is active, bristles 28 take on a negative charge. At the same time, positively charged ions are transferred to the teeth 29 via the conducting pathway through a moist hand holding the positively charged handle 24. Thus, the polarity of the teeth 29 are changed from negative to positive. The now positively charged teeth repel the positively charged plaque ions, which are furthermore attracted to the negatively charged toothbrush bristles 28. Moist finger contact is essential to maximize ionic transfer.

Similar to the prior art iontophoretic dental tray depicted in Fig. 1, the circuit is completed only through the body of the user, resulting in electrochemical depolarization, which decreases the ionization and thus the plaque transfer within the system.

There is thus provided an improved system for enhanced fluoride treatment of teeth, which is designed to increase ionization of fluoride by directing a circuit independently of the body, and by using preparatory enhancements, as will be described in further detail hereinbelow.

Reference is now made to Fig. 3, which is an illustration of a pre-treatment tray 30. Pre-treatment tray 30 is designed to hold an activation solution for immersion of the teeth prior to treatment with fluoride solution. The activation solution will be described in further detail hereinbelow. Pre-treatment tray 30 has an upper portion 32 and a lower portion 34. Upper portion 32 includes a sealing lip 36, which is designed to contact the gums, thus sealing the tray onto the gums and substantially preventing leaking out of activation solution. Lower portion 34 includes a space 35 preferably having an absorbent material 38 therein. In a preferred embodiment, absorbent material 38 is a high density sponge. Absorbent material 38 is designed to absorb the activating solution, and further prevent leakage. In a preferred embodiment, a pre-measured amount of activating solution is introduced directly into absorbent material 38 prior to placing pre-treatment tray 30 on the teeth, so as to ensure that a suitable amount of solution is used for full immersion of the teeth without excessive solution being present. In an alternative embodiment, space 35 is empty.

In one embodiment, pre-treatment tray 30 is comprised of a plastic material which can be heated and formed on the teeth and gums during application. Alternatively, an adhesive material which is biocompatible (such as, for example, beeswax) may be added to sealing lip 36 to further ensure sealing.

The activating solution prepares the surface of the teeth to make them receptive to the ionic changes that are designed to occur during operation of the apparatus or device described herein. The activating solution includes a metal or metallic salt, such as silver nitrate, palladium hydroxide, palladium chloride, copper chloride or any other suitable activator. In a preferred embodiment the activating solution is applied through teeth immersion in the application pre-treatment tray 30 of Fig. 3. In yet another less preferred embodiment, the solution is prepared as a rinse, similar to mouthwash. The metal or metal salts provide a preparatory surface on the teeth, wherein the tooth surface adsorbs the material, for example, silver or its ion, and thereby the electric conductivity of the tooth surface for the flowing current is increased (or the electric resistance of the treated tooth surface to the flowing current is decreased). This process facilitates substitution of the hydroxyl group bound to apatite with the fluoride present in the medium. Once the tooth surface is prepared, the metal is rinsed out, and subsequently, a treatment device (tray) is applied to the teeth, as described below.

Reference is now made to Fig. 4, which is an isometric view of a device 41 for ion exchange fluoride treatment in accordance with a preferred embodiment of the present invention. Device 41 is a tray designed to hold a fluoride solution for immersion of teeth therein. Device 41 is of a shape similar to typical dental trays, including a back curved wall 43 and a front curved wall 45 joined together at end sections 47. Back curved wall 43 includes an electrode 49, which is the negative electrode, or the anode. Electrode 49 comprises a thin, flexible metal strip attached to an inner portion of back curved wall 43. In a preferred embodiment, electrode 49 is comprised of stainless steel. In alternative embodiments, electrode 49 is comprised of any inert material, such as, platinum, gold, or any other suitable conducting metal. Spacers 51 are positioned along electrode 49, and are designed to preclude contact between electrode 49 and teeth positioned within device '41, while providing contact between the fluoride solution within device 41 and electrode 49. Spacers 51 are comprised of insulating materials, such as plastics. Front curved wall 45 includes contact electrodes 53, which are positive electrodes, or cathodes, and are designed to be placed in direct contact with the front of teeth within device 41. Contact electrodes are attached to front curved wall 45 by a spring-like mechanism, ensuring contact between contact electrodes 53 and the teeth. Preferably, at least three contact electrodes 53 are used, each one designed to contact a different tooth. The contact area between contact electrodes 53 and the teeth is minimized. In a preferred embodiment, device 41 further includes a sealing lip such as the one described with reference to pre-treatment tray 30 of Fig. 3. In alternative embodiments, device 41 is comprised of heatable plastics, which can be formed on the teeth and gums during placement. An adhesive or a repelling substance such as Vaseline™ may also be used to further ensure sealing.

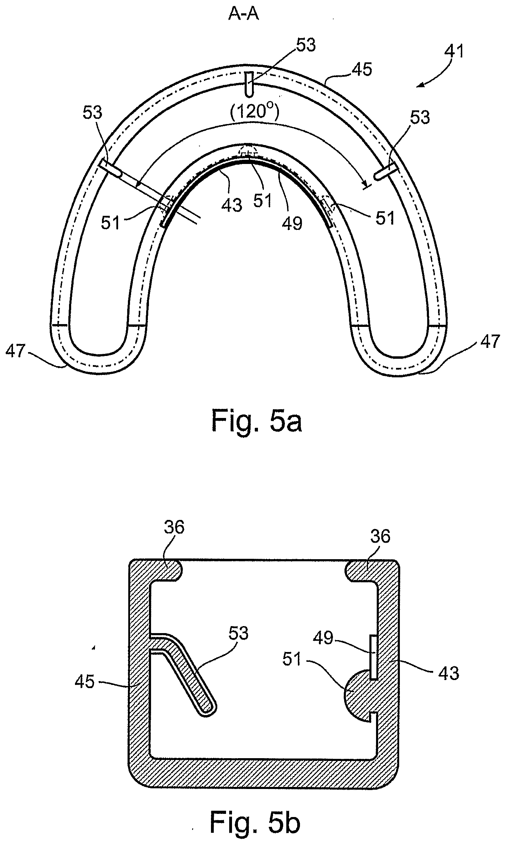

Reference is now made to Figs. 5a and 5b, which are cross sectional illustrations of device 41 along lines A-A and B-B, respectively. As shown in Fig. 5a, spacers 51 may contact the back of the teeth, while contact electrodes 53 are designed to contact the front of the teeth. As shown in Fig. 5b, spacers 51 are fixed, and contact electrodes are attached to front curved wall 45 by a spring-like mechanism. In the embodiment shown in Fig. 5b, the spring-like nature is accomplished by placing contact electrode 53 at an angle. Alternatively, actual springs may used. In a preferred embodiment, three contact electrodes 53 contact three different teeth. The contact area of the electrodes is minimal so as to induce current into the teeth while avoiding an electrolysis response.

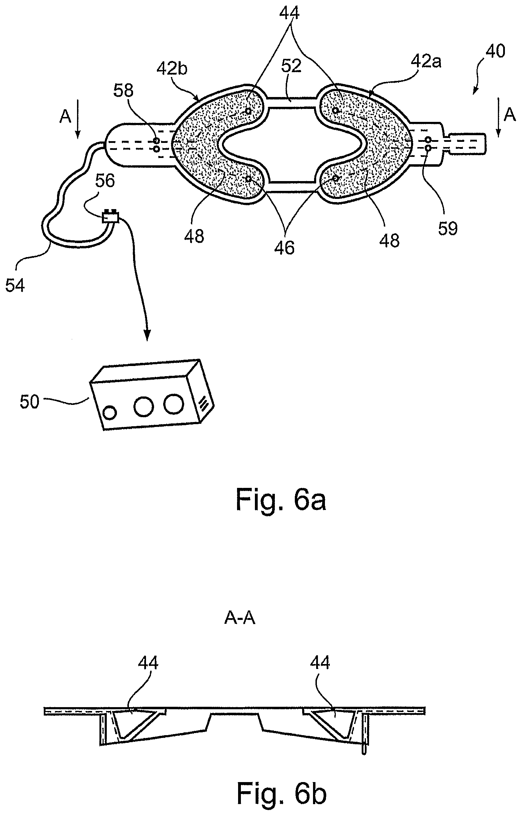

Reference is now made to Figs. 6a and 6b, which are an illustration and a cross-sectional view, respectively, of an apparatus 40 for fluoride treatment in accordance with another embodiment of the present invention. Apparatus 40 includes a set of trays 42, including an upper tray 42a and a lower tray 42b, each of which has wells 44 for holding a fluoride gel composition. Trays 42 have plugs and electrodes 46 positioned therein, which are connected via conducting wires 48 to a power supply 50. Electrodes are comprised of inert material, such as stainless steel, platinum, gold, or any other suitable conducting metal. In a preferred embodiment, trays 42 are comprised of a non-conducting material, such as a biocompatible plastic material. It should be noted that any biocompatible plastic material which does not react with fluoride may be used. Trays 42 and wells 44 are sized in accordance with standard sized dental trays. In one embodiment the upper and the lower trays 42 are connected to each other via a folding bridge 52, which allows both trays 42 to be placed on the teeth simultaneously by folding the trays back in the area of folding bridge 52. An electrical connector 54 connects electrodes 48 to power source 50 via a plug 56, or via any other suitable means. Plug 56 is designed to fit into power source 50. Furthermore, upper and lower trays 42 are connectable to one another via a socket 58 and socket connector 59. Socket 58 is located on an outer rim of one of trays 42, such that when trays 42 are folded at folding bridge 52, socket connector 59 connects the two trays 42 and completes the circuit. In another embodiment, the trays 42 are not connected to each other.

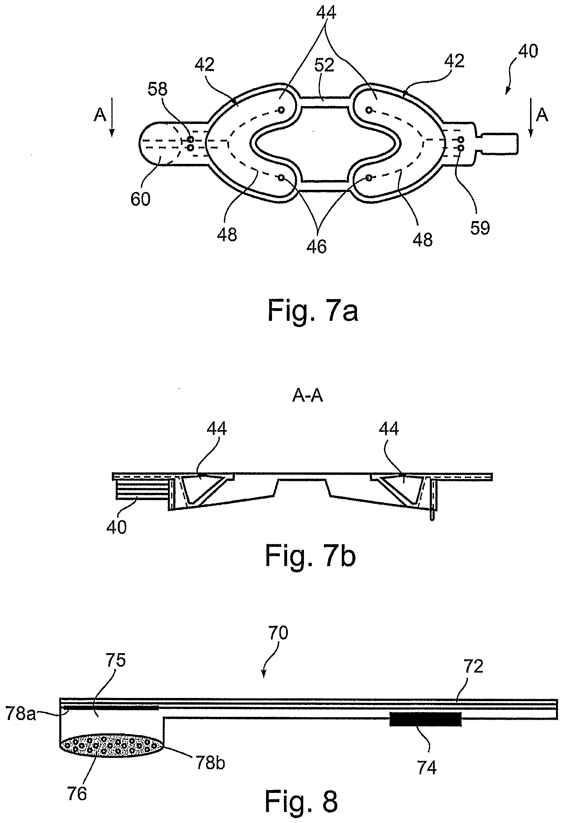

In one embodiment, as shown in Fig. 6a, device 41 is connected to a regular power supply, with a standard AC/DC transformer. In another embodiment, rechargeable batteries (1-12 volt) are used. In yet another embodiment, shown in Figs. 7a and 7b, a disposable battery is embedded in the device, allowing it to be more easily transportable.

It should be readily apparent that in all of the described embodiments, the circuit runs through apparatus 40, and not through the body of the user. In this way, fluoride ionization can be increased, thus enhancing fluoride uptake by the teeth,

In one embodiment, different sized pre-treatment trays 30 and devices 41 are provided for different sized individuals.

Reference is now made to FIG. 8, which is an illustration of an ionic toothbrush 70, in accordance with an embodiment not covered by the present invention. Toothbrush 70 includes a handle 72 having a battery 74 placed therein, and a head portion 75 at an opposite end thereof. Head portion 75 includes a bristle portion 76 on a lower end thereof, and further has two electrodes 78 - a cathode 78a on an upper end and an anode 78b in an area of the bristles. A circuit is completed through cathode 78a, the tooth which is in contact with bristle portion 76, and anode 78b. Toothbrush 70 can be used with a fluoride gel as in the dental trays above to increase fluorination on the teeth. Alternatively, toothbrush 70 can be used to decrease the plaque on the tooth surface. The circuit of the toothbrush 70 does not run through the body of an individual.

The fluoride gel is provided in the form of a compound, and is suitable for donating fluoride ions. Examples of such compounds include but are not limited to sodium fluoride, lithium fluoride, amino fluoride, tin fluoride, a combination of fluoride donor compounds, or any other suitable compound which is readily ionizable.

A method of pre-treating teeth for fluoride uptake used in accordance with the apparatus described above is as follows, in accordance with one embodiment of the present invention. Initially, teeth are thoroughly cleaned, either by professional cleaning or using a regular toothbrush. Next, teeth are rinsed with distilled water, followed by a rinse with hydrogen peroxide, and another rinse with distilled water. Hydrogen peroxide rinse is preferably a diluted solution (18% hydrogen peroxide), obtained by mixing equal amounts of commercial hydrogen peroxide (36%) and distilled water. A metal catalyst solution is prepared by dissolving different amounts of metal or metal salt solutions such as silver, copper or palladium in an amount of distilled water such that the concentration of the metal is in the range of 0.01-3% by weight. In preferred embodiments, the metal solutions include silver nitrate, palladium hydroxide, palladium chloride, copper chloride salts or any other suitable activator. In one example, 100 mg of silver nitrate is dissolved in 10 ml distilled water. In yet another example 50 mg of copper chloride is dissolved in 10 ml of distilled water. Pre-treatment tray 30 is placed on the teeth, preferably in such a way that all of the teeth are in contact with the sponge. Sealing lip 36 is placed in contact with the gums to seal tray 30 and prevent leaking of solution into the mouth. A pre- , measured amount of the metal catalyst solution is then introduced into tray 30 by injection or any other filling procedure such that full contact between the teeth and the solution is obtained, for approximately 1 minute. Alternatively, a known amount of metal catalyst solution is placed into pre-treatment tray 30 prior to placing tray 30 in the mouth. After pretreatment, tray 30 is removed from the mouth, taking care to avoid spilling into the mouth. Teeth are then rinsed with distilled water again.

After, pretreatment, fluoride treatment is commenced. A fluoride donor solution is prepared as follows. The following description of the preparation of the fluoride donor solution includes sodium fluoride, but it should be readily apparent that this is merely exemplary, and that the fluoride solution is not limited to this compound. Sodium fluoride solution in a range of 1-5% is prepared by dissolving an appropriate amount of sodium fluoride salt in distilled water. Sodium fluoride solution is incorporated into a gel, such as alginate and added to an apparatus of the present invention, either by filling the trays of the dental tray embodiment, or by coating the bristles of a toothbrush such as the one described above. In a preferred embodiment, a commercial fluoride gel having a 2.5% concentration is combined with 0.5 grams of sodium chloride (used as electrolyte) dissolved in 25 ml of distilled water, providing a total percentage of fluoride ions as 1.25%. In an alternative embodiment, 25 grams of Elmex™ Gel is mixed with a solution of 0.7 grams of sodium fluoride, followed by combination with 0.5 grams of sodium chloride and dissolution in 25 ml of distilled water. A power supply of 0-12 volts is used. The electric circuit is then activated with a voltage of 1-12 volts. In a preferred embodiment, the voltage is in the range of 3 to 9 volts, and most preferably is approximately 6 volts. The circuit remains open for a predetermined period of time, in the range of 5-20 minutes. The current does not exceed 30 mA.

In one embodiment, a kit is provided, wherein a suitable sized pre-treatment tray 30, a suitable sized device 41, and a compatible amount of pre-measured activation solution and fluoride donor solution are provided. Different kits may be provided for different sized and aged individuals.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLE

Reference is now made to the following example, which together with the above description, illustrate the invention in a non limiting fashion.

Fresh extracted teeth were washed with hydrogen peroxide solution (18%) for decontamination prior to use. Teeth were split into five groups, as follows:
Group A (control, n=3): No pretreatment, No gel treatment
Group B (n=3): No pretreatment, Teeth immersed for 10 minutes in prepared fluoride gel, no current.
Group C (n=3): Yes pretreatment (immersed in 1% silver nitrate solution for 1 minute), Teeth immersed for 10 minutes in prepared fluoride gel, no current.
Group D (n-3): No pretreatment, Teeth immersed for 10 minutes in prepared fluoride gel with electric current.
Group E (n=3): Yes pretreatment (immersed in 1% silver nitrate solution for 1 minute), Teeth immersed for 10 minutes in prepared fluoride gel with electric current.

All teeth were then rinsed with water and placed in 5% lactic acid solution, having a pH of 1.9, and left in lactic acid solution for different time periods, ranging from 15 to 4500 minutes. At each time period, the tooth was rinsed with distilled water and tested by scratching with a sharp metal tool. If the scratching resulted in a scratch mark, the tooth was considered to have failed the test. If no scratch mark resulted, the tooth was considered to have passed the test.

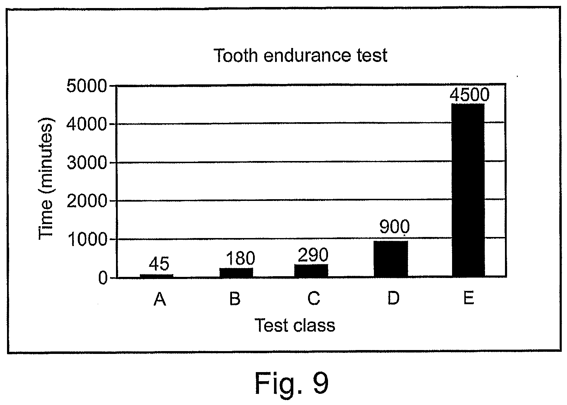

Results for the above experiment are summarized in Fig. 9, in bar graph format.

To summarize, the teeth from Group A (control) failed after an average of 45 minutes of exposure. Teeth from Group II failed after 2-3 hours of exposure. Teeth from Group III failed after 4-5 hours of exposure. Teeth from Group IV failed after approximately 15 hours of exposure. Teeth from Group V lasted at least 75 hours.

The above results show that teeth with pretreatment and current are significantly stronger than all other combinations.

Based on the above results, it is clear that pretreatment of teeth with a metal or metal salt solution, followed by connection to a circuit in an electrolyte having fluoride or a fluoride releasing substance, enhances and accelerates fluorination of the teeth, which results in increased physical resistance of the teeth against decay. This procedure can be accomplished with either a dental device or a toothbrush, as described above. The treatment is relatively short, and low in cost. It may be performed by any clinician, such as a dentist or hygienist.

In addition to the fluorination process described above, the present invention can be used to enhance other dental treatments as well. For example, tooth whitening procedures may be enhanced using the proposed methods and devices. Furthermore, strengthening of the gums can be accomplished.

## Claims

1. A device (41) for dental treatment, the device comprising:
an applicator (43, 45, 47) for applying a substance to a tooth, said applicator having a first end and a second end;
a first electrode (49) attached to a first end of said applicator;
a second electrode (53) attached to said second end of said applicator, wherein said first electrode and said second electrode are configured to produce a current flow through said applicator; and
an ionizable substance for placement within said applicator, wherein said ionizable substance is configured to undergo ionization upon application of said current flow;
**characterized in that** said applicator is a dental tray (41).

2. The device of claim 1, wherein said ionizable substance is a fluoride donor compound.

3. The device of claim 2, wherein said fluoride donor compound is enriched with sodium fluoride, lithium fluoride, amino fluoride, tin fluoride, or a combination of fluoride donor compounds.

4. The device of claim 1, wherein said first end is a back curved wall and said first electrode is an anode.

5. The device of claim 4, wherein said anode is a flat metal strip.

6. The device of claim 5, further comprising spacers on said anode to separate said anode from said tooth.

7. The device of claim 6, wherein said spacers are comprised of a biocompatible plastic material.

8. The device of claim 1, wherein said second end is a front wall and said second electrode is a cathode.

9. The device of claim 8, wherein said cathode further comprises a spring mechanism.

10. The device of claim 8, wherein said cathode includes three cathodes, and wherein each of said three cathodes is configured to contact a separate tooth at a contact area on said tooth, wherein said contact area is minimized.

11. The device of claim 1, wherein said dental tray further comprises a sealing lip.

12. The device of claim 1, further comprising a power supply, wherein said power supply is an external power source, a rechargeable battery, or a disposable battery embedded within said applicator.

## Patentansprüche

1. Vorrichtung zur Zahnbehandlung, wobei die Vorrichtung aufweist:
einen Applikator (43, 45, 47) zum Auftragen einer Substanz auf einen Zahn, wobei der Applikator ein erstes und ein zweites Ende hat;
eine erste Elektrode (49), die am ersten Ende des Applikators angebracht ist;
eine zweite Elektrode (53), die am zweiten Ende des Applikators angebracht ist, wobei die erste und die zweite Elektrode so konfiguriert sind, dass sie einen Stromfluss durch den Applikator erzeugen; und
eine ionisierbare Substanz zum Einbringen in den Applikator, wobei die ionisierbare Substanz so konfiguriert ist, dass sie bei Beaufschlagung mit dem Stromfluss eine Ionisierung erfährt;
**dadurch gekennzeichnet, dass** der Applikator eine Zahnschale (41) ist.

2. Vorrichtung nach Anspruch 1, wobei die ionisierbare Substanz eine Fluoriddonorverbindung ist.

3. Vorrichtung nach Anspruch 2, wobei die Fluoriddonorverbindung mit Natriumfluorid, Lithiumfluorid, Aminfluorid, Zinnfluorid oder einer Kombination aus Fluoriddonorverbindungen angereichert ist.

4. Vorrichtung nach Anspruch 1, wobei das erste Ende eine nach hinten gekrümmte Wand und die erste Elektrode eine Anode ist.

5. Vorrichtung nach Anspruch 4, wobei die Anode ein Flachmetallstreifen ist.

6. Vorrichtung nach Anspruch 5, die ferner Abstandsstücke an der Anode aufweist, um die Anode vom Zahn zu trennen.

7. Vorrichtung nach Anspruch 6, wobei die Abstandsstücke aus einem biokompatiblen Kunststoffmaterial bestehen.

8. Vorrichtung nach Anspruch 1, wobei das zweite Ende eine vordere Wand und die zweite Elektrode eine Kathode ist.

9. Vorrichtung nach Anspruch 8, wobei die Kathode ferner einen Federmechanismus aufweist.

10. Vorrichtung nach Anspruch 8, wobei die Kathode drei Kathoden umfasst, und wobei jede der drei Kathoden so konfiguriert ist, dass sie einen separaten Zahn an einem Kontaktbereich des Zahns berührt, wobei der Kontaktbereich minimiert ist.

11. Vorrichtung nach Anspruch 1, wobei die Zahnschale ferner eine Dichtlippe aufweist.

12. Vorrichtung nach Anspruch 1, die ferner eine Energiequelle aufweist, wobei die Spannungsquelle eine externe Energiequelle, eine wiederaufladbare Batterie oder eine in dem Applikator eingebettete Einweg-Batterie ist.

## Revendications

1. Dispositif (41) pour un traitement dentaire, le dispositif comprenant :
un applicateur (43, 45, 47) pour appliquer une substance à une dent, ledit applicateur ayant une première extrémité et une deuxième extrémité ;
une première électrode (49) fixée à une première extrémité dudit applicateur ;
une deuxième électrode (53) fixée à ladite deuxième extrémité dudit applicateur, dans lequel ladite première électrode et ladite deuxième électrode sont configurées pour produire un flux de courant à travers ledit applicateur ; et
une substance ionisable pour placement à l'intérieur dudit applicateur, dans lequel ladite substance ionisable est configurée pour subir une ionisation à l'application dudit flux de courant ;
**caractérisé en ce que** ledit applicateur est un plateau dentaire (41).

2. Dispositif selon la revendication 1, dans lequel ladite substance ionisable est un composé donneur de fluorure.

3. Dispositif selon la revendication 2, dans lequel ledit composé donneur de fluorure est enrichi avec du fluorure de sodium, du fluorure de lithium, de l'aminofluorure, du fluorure d'étain, ou une combinaison de composés donneurs de fluorure.

4. Dispositif selon la revendication 1, dans lequel ladite première extrémité est une paroi incurvée vers l'arrière et ladite première électrode est une anode.

5. Dispositif selon la revendication 4, dans lequel ladite anode est une bande métallique plate.

6. Dispositif selon la revendication 5, comprenant en outre des espaceurs sur ladite anode pour séparer ladite anode de ladite dent.

7. Dispositif selon la revendication 6, dans lequel lesdits espaceurs sont composés d'un matériau plastique biocompatible.

8. Dispositif selon la revendication 1, dans lequel ladite deuxième extrémité est une paroi avant et ladite deuxième électrode est une cathode.

9. Dispositif selon la revendication 8, dans lequel ladite cathode comprend en outre un mécanisme à ressort.

10. Dispositif selon la revendication 8, dans lequel ladite cathode inclut trois cathodes, et dans lequel chacune desdites trois cathodes est configurée pour contacter une dent séparée au niveau d'une zone de contact sur ladite dent, dans lequel ladite zone de contact est minimisée.

11. Dispositif selon la revendication 1, dans lequel ledit plateau dentaire comprend en outre une lèvre d'étanchéité.

12. Dispositif selon la revendication 1, comprenant en outre une alimentation électrique, dans lequel ladite alimentation électrique est une source d'énergie externe, une batterie rechargeable, ou une batterie jetable intégrée à l'intérieur dudit applicateur.
